# EUROPEAN PATENT SPECIFICATION

(11) **EP 2 033 602 B1**
(45) Date of publication and mention of the grant of the patent: **06.11.2013**
(21) Application number: 08171809.0
(22) Date of filing: 27.03.2003
(51) Int. Cl.: A61F 2/958

(54) **Endolumenal device for delivering and deploying an endolumenal expandable prosthesis**
Vorrichtung zur Einführung und zum Einsetzen einer ausdehnbaren Endoluminalprothese
Dispositif pour l'introduction et la pose d'une endoprothese endoluminale

(30) Priority: 22.04.2002 IT MI20020860
(43) Date of publication of application: 11.03.2009
(62) Divisional of application: 03708427.4
(73) Proprietor: EVR Medical S.à.r.l, 1420 Luxembourg (LU)
(72) Inventor: Lualdi, Alessandro, I-20010, Marcallo con Casone, Milano (IT)
(74) Representative: Crippa, Paolo Ernesto

(56) References cited:
- EP-A- 0 891 751
- WO-A-00/74595
- WO-A-01/60284
- US-A- 6 099 497
- US-A1- 2001 004 706

## Description

The subject of the present invention is an endolumenal device for delivering and deploying an endolumenal expandable prosthesis. In particular, the present invention refers to a device comprising an elongated body having a proximal end portion and a distal end portion. The distal end portion of said elongated body comprises expansion means removably engageable with the endolumenal expandable prosthesis and adapted to adjust said prosthesis from a radially collapsed condition to a radially expanded condition. Said device also comprises a guidewire tracking means extending at least partially along said elongated body.

### Endolumenal devices of these type are known from WO2001/60284 and US 6 099 497.

As is known, devices of the type described above are used for delivering and deploying, meaning in particular fitting or implanting, prostheses or stents endolumenally within conduit systems, such as for example vessels carrying body fluids and, in particular, lumens in the bodies of human beings and animals. Said vessels for the transportation of fluids are, for example, arterial blood vessels, such as coronary, mesenteric, peripheral and cerebral arteries, veins or gastrointestinal tracts.

Using the abovementioned devices it is possible, for example, to implant endolumenal prostheses, or stents, in a vessel in which arteriosclerotic plaque, or arteriostenosis, has partially or completely occluded the lumen. Said prosthesis forms a radial support for the surrounding wall of the lumen and prevents it partially or completely occluding again, once it has been dilated by the expansion means. These operations are carried out using known angioplasty techniques. Techniques of this type are, for example, described in the publication "The New Manual of Interventional Cardiology" edited by Mark Freed, Cindy Grines and Robert D. Safian, Division of Cardiology at William Beaumont Hospital, Royal Oak, Michigan; Physicians' Press 1996.

It is also known that the use of said techniques of angioplasty for percutaneous or endolumenal revascularization is increasingly used as an alternative to standard by-bass and thromboendoarteriectomy surgical procedures, not only in cases with acute complications, such as dissection of the vessel and acute occlusion, but also in the elective treatment of coronary and systemic arteriosclerotic lesions.

The widespread use of these techniques is considerably limited by the significant difficulties presented by the known endolumenal devices when they are used on vascular ramifications or bifurcations of the system of conduits (bifurcation lesions).

It is known that operations on bifurcation lesions are frequently subject to procedural failures and acute complications, because the known devices cause occlusion of that branch of the bifurcation which originates near the area in which the prosthesis is fitted.

In particular, due to the activation of the expansion means in a first branch of the bifurcation, the atheromatous material of the plaques is protruded and displaced until it obstructs the ostium of a second branch of the bifurcation, (a problem known as snow-plow or plaque-shifting).

Due to the abovementioned snow-plough or plaque-shifting, the ostium of the occluded branch must again be rendered accessible, or regained, by re-introducing a guidewire through a barrier consisting of the plaque previously protruded and displaced until it obstructed the lumen.

In other words, it is necessary, following the implanting of the first prosthesis, to insert a second guidewire and a second prosthesis into the occluded branch, passing through the meshes or struts of the first prosthesis. Even when it is possible to regain access to the occluded branch, the operation becomes extremely lengthy and, in any case, the results depend very much on the experience of the surgeon.

Where the above-described bifurcation lesions are present it is therefore essential that the operation is carried out in highly qualified centres, fully equipped for cardiac surgery, that may be called upon urgently in the case of damage following occlusions caused during the endolumenal operation and lack of success in regaining the ostium.

Due to the abovementioned difficulties, the use of stents with wide apertures to allow the passage of the prosthesis and the introduction of a guidewire into the branches has been proposed. However, these wide apertures can give rise to an increase in prolapse of plaque through the meshes and, therefore, imperfect vascularization and increased probability of re-stenosis.

One alternative that has been proposed is the simultaneous use of two devices fitted with expansion means for the simultaneous insertion of two stents in each of the branches of the bifurcation (paired or kissing devices), or of a single bifurcated stent.

This known solution however is very bulky and difficult to manoeuvre and can only be used in large vessels and in neighbouring portions. In other words, it is impossible to use this known solution in peripheral branches, where the formation of atheromes or arteriosclerotic plaques is more likely. Furthermore, in order to insert the known paired devices it is necessary to use large-diameter guide catheters. The greater bulk of the paired devices occludes the vessel during insertion causing ischemia during the procedure and making it impossible to inject a contrast medium which is useful for visualizing the path for the correct positioning, first of the guidewire and then of the endolumenal devices fitted with the prosthesis.

The use of paired devices also lacks versatility, above all in the case of a single bifurcated stent, since the three vascular segments which make up the bifurcation - the proximal principal vessel, the principal vessel distal to the bifurcation and the secondary vessel, or side branch - may be of very different bores with lesions of varying lengths. It is therefore impossible at present to prepare a range of bifurcated stents which can be adapted to all the possible anatomical and pathological variables. It must also be noted that these bifurcated stents, of fixed dimensions, often occlude other branches near the bifurcation lesions, with consequent ischemia or incomplete revascularization.

It is therefore evident that not all bifurcation lesions, and in particular coronary bifurcation lesions, can be dealt with percutaneously.

The above considerations show that the need for an endolumenal device for delivering and deploying an endolumenal expandable prosthesis, which can reach both the branches of a bifurcation safely and rapidly, is widely felt. A need is likewise felt to be able to fit endolumenal prostheses which are morphologically adaptable to the anatomy and to the pathology of the proximal and distal portions of the branches of the bifurcation. In other words, it is desirable to be able to deal with all types of lesions using a single endolumenal device, of the type described above, capable of adapting to a vast range of vessel diameters and lesions of any length. Said endolumenal device must also ensure the accurate deployment of the prosthesis, guaranteeing ample coverage of the bifurcation in order to prevent protrusion of plaque between the various prostheses fitted and the formation of re-stenosis.

Therefore, the object of this invention is to devise and make available an endolumenal device of the type specified above, which will meet all the abovementioned requirements and, at the same time, make it possible to avoid all the pitfalls outlined.

This object is achieved by means of an endolumenal device according to Claim 1.

Further characteristics and advantages of the endolumenal device according to the invention will become evident from the description that follows of some preferred embodiments, which are given purely by way of indication and without implying any limitation, with reference to the enclosed drawings, in which:
- Figure 1 shows a partially sectioned perspective view of an endolumenal device fitted with a prosthesis;
- Figures 2 and 3 show a view from beneath, and a side view, of a detail of the device of Figure 1;
- Figures 4 and 4a show the enlarged section on IV-IV through the device of Figure 2;
- Figure 5 shows an end view along the arrow V of the endolumenal device of Figure 3;
- Figures 6a and 6b show a partially sectioned view of the device of Figure 1 during two stages of use;
- Figures 7 and 8 show a view from beneath, and a side view, of a detail of an endolumenal device;
- Figure 9 shows the enlarged section on IX-IX through the device of Figure 7;
- Figure 10 shows a front view along the arrow X of the device of Figure 8;

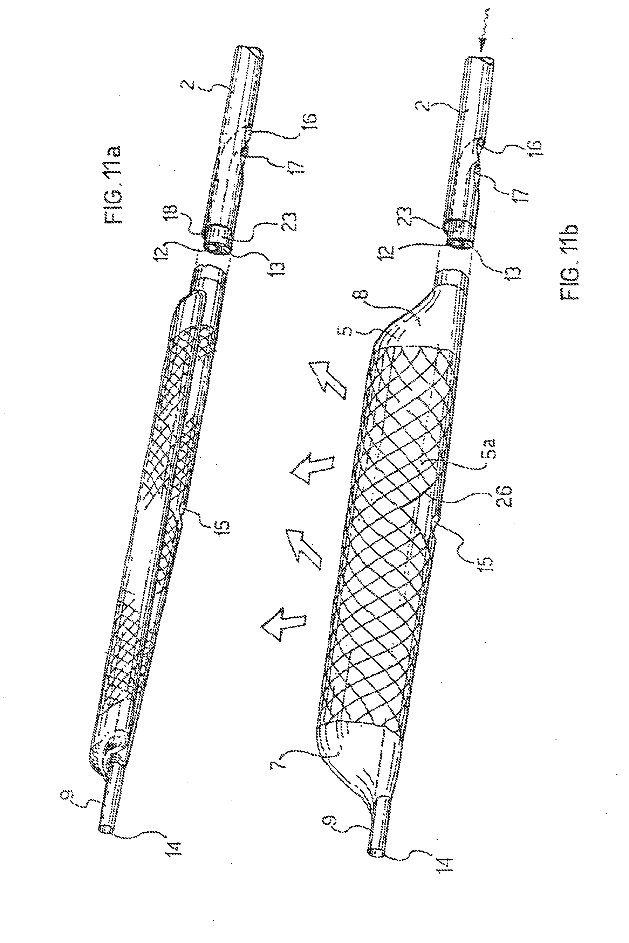

- Figures 11a and 11b show a partially sectioned perspective view of the device of Figure 7 during two stages of use;
- Figures 12 to 17c show a section through a 'T bifurcation' during eight stages in the implanting of endolumenal prostheses;
- Figures 17d and 17e show in section two alternative stages in the implanting of prostheses in the bifurcation shown in Figure 17c;
- Figures 18 to 23 show a section through a 'Y bifurcation' during six stages in the implanting of endolumenal prostheses;

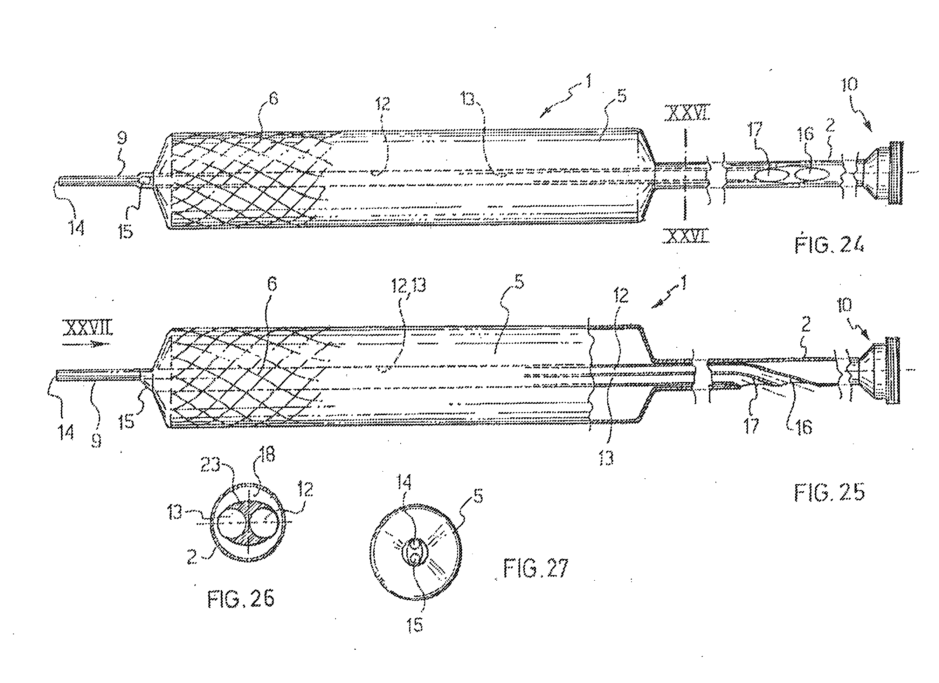

- Figures 24 and 25 show a view from beneath, and a side view, of an endolumenal device;
- Figure 26 shows an enlarged section on XXVI-XXVI through the device of Figure 24;
- Figure 27 shows a view along the arrow XXVII of the device of Figure 25;
- Figures 28 and 29 show a view from beneath, and a side view partially sectioned, of details of an endolumenal device;
- Figure 30 shows a view along the arrow XXX of the device of Figure 29;
- Figure 31 is a perspective view of a portion of a possible embodiment of an endolumenal device sectioned at two points along its longitudinal length;
- Figure 32 is a longitudinal section through a possible embodiment of the device shown in Figure 31;
- Figure 33 is a perspective view of the device shown in Figure 32, sectioned on XXXIII-XXXIII as shown in Figure 32;
- Figure 34 is a longitudinal section through an embodiment of the device shown in Figure 31;
- Figure 35 is a perspective view of the device shown in Figure 34, sectioned on XXXV-XXXV as shown in Figure 34;

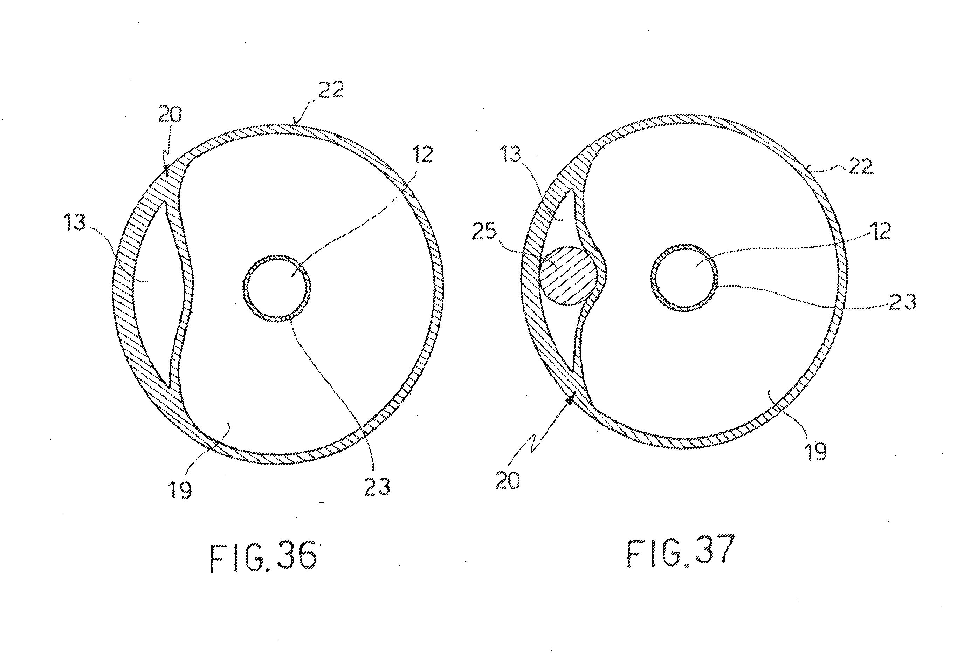

- Figure 36 shows an enlarged cross section through the device shown in Figure 32 or 34 on XXXVI-XXXVI as shown in Figure 32 or 34;
- Figure 37 shows the enlarged cross section of Figure 36 illustrating the device into which a guidewire has been inserted; and
- Figure 38 is a cross section through an endolumenal device in another possible embodiment.

With reference to the above figures, the number 1 indicates as a whole an endolumenal device for delivering and deploying an endolumenal expandable prosthesis or inflatable catheter. For example, the abovementioned device is suitable for deploying an expandable endolumenal prosthesis with a bifurcation having one principal conduit and at least one secondary conduit.

Said endolumenal device includes an elongated body 2 having a distal end portion 3 and a proximal end portion 4. For example, said elongated body 2 is between 100 cm and 160 cm in length, and preferably between 115 cm and 140 cm. The distal end portion 3 includes expansion means, 5, which can be removably engaged with an endolumenal expandable prosthesis 6. Said expansion means 5 can adapt said prosthesis 6 from a radially collapsed to a radially expanded position, in a manner which will be described in greater detail below. The expansion means 5 include a distal portion 7 of the expansion means, a proximal portion 8 of the expansion means and a central portion 5a of the expansion means to which the prosthesis 6 can be attached. In one embodiment, the distal portion of the elongated body 3 extends beyond the expansion means 5 in an apical portion 9. At the proximal end of the proximal end portion 4 of the elongated body 2, there are means 10 for connecting the endolumenal device 1 to a device of a type known per se for the controlled activation of the expansion means 5.

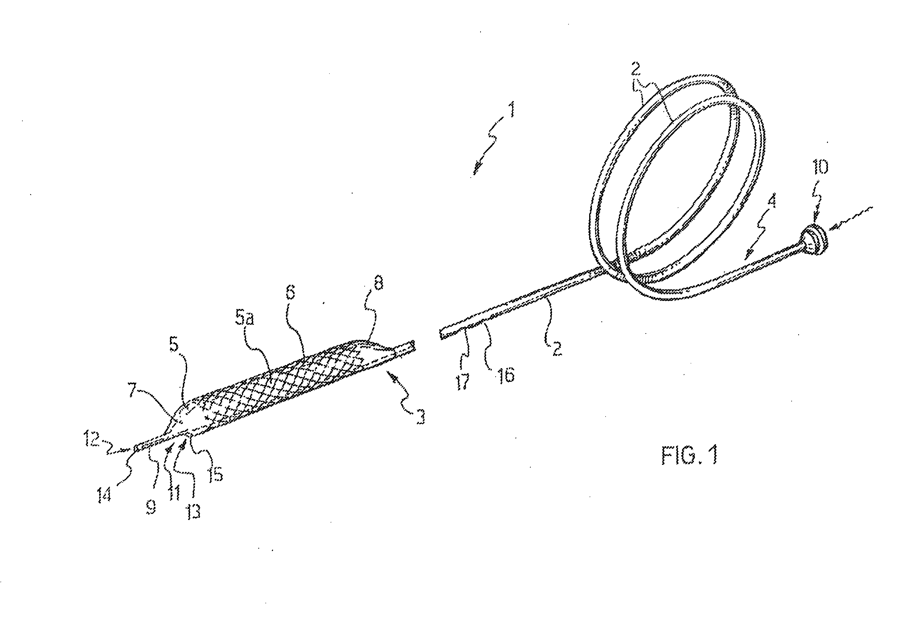

The endolumenal device 1 also includes guidewire tracking means 11 which extend at least partially along the elongated body 2. For example, said means 11 extend along the distal end portion 3 of the elongated body 2 close to the expansion means 5 (Figure 1).

Advantageously, the active portion of the expansion means may be longitudinally attached to the elongated body to expand the prosthesis eccentrically to one side with respect to the elongated body so that the other side of the elongated body is left free of said expanded active portion.

Advantageously, said guidewire tracking means 11 comprise a first guidewire lumen 12 which extends at least partially inside the elongated body 2.

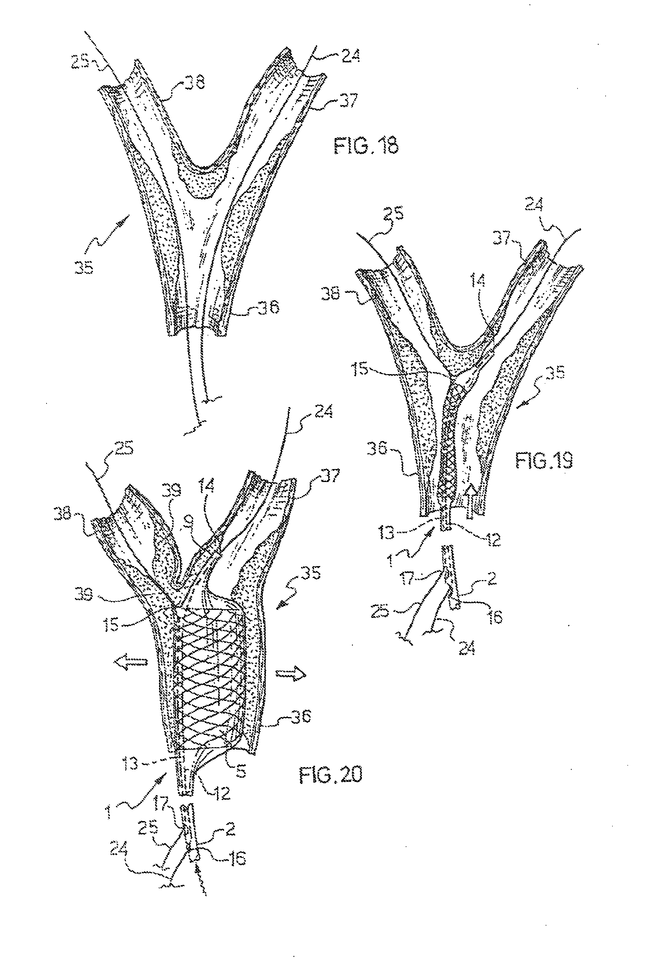

A first guidewire lumen 12 and a second guidewire lumen 13 extend completely inside the elongated body 2. Distal ports 14, 15 and proximal ports 16, 17 make said first and second lumens 12, 13 able to receive guidewires 24, 25 (Figure 19).

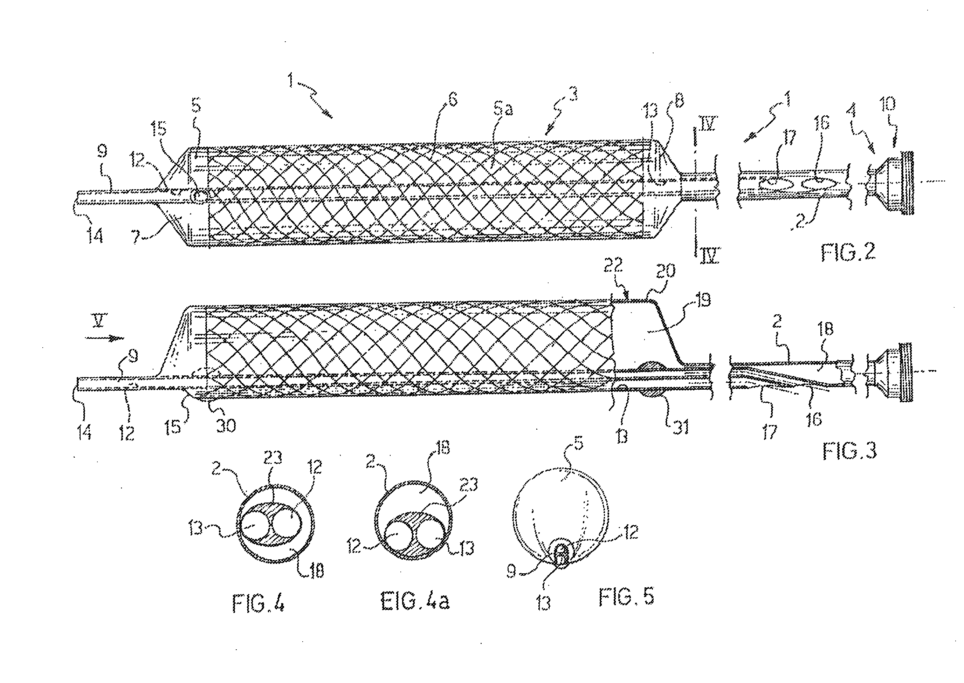

The first guidewire lumen 12 extends entirely inside the elongated body 2. The first guidewire lumen 12 may extend between a distal port 14 and a proximal port 16, connecting with the walls of the elongated body 2 only in the vicinity of the respective distal and proximal ports (Figure 5).

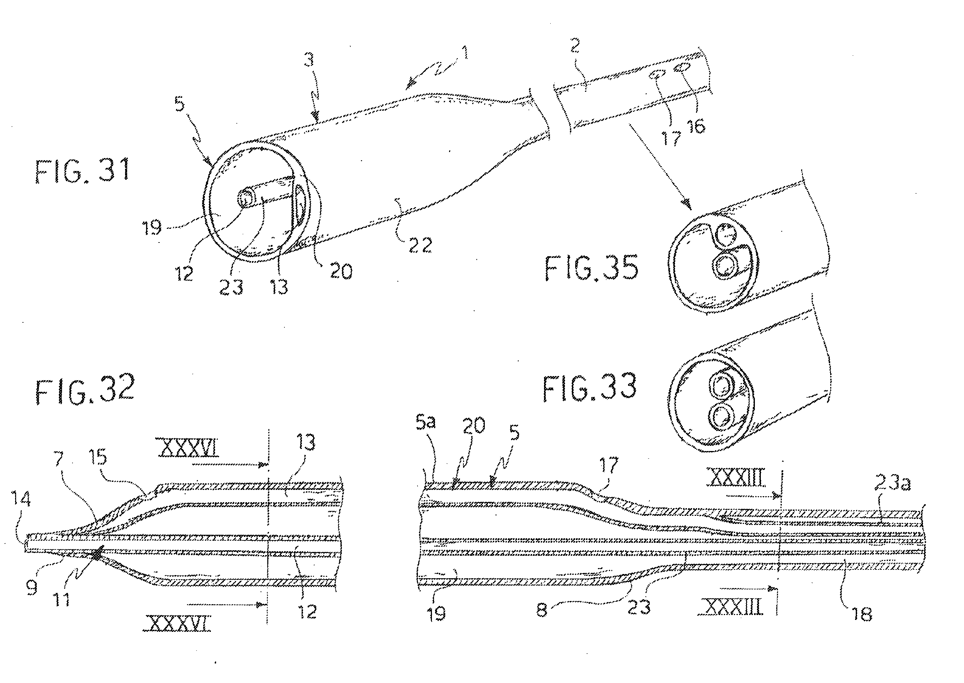

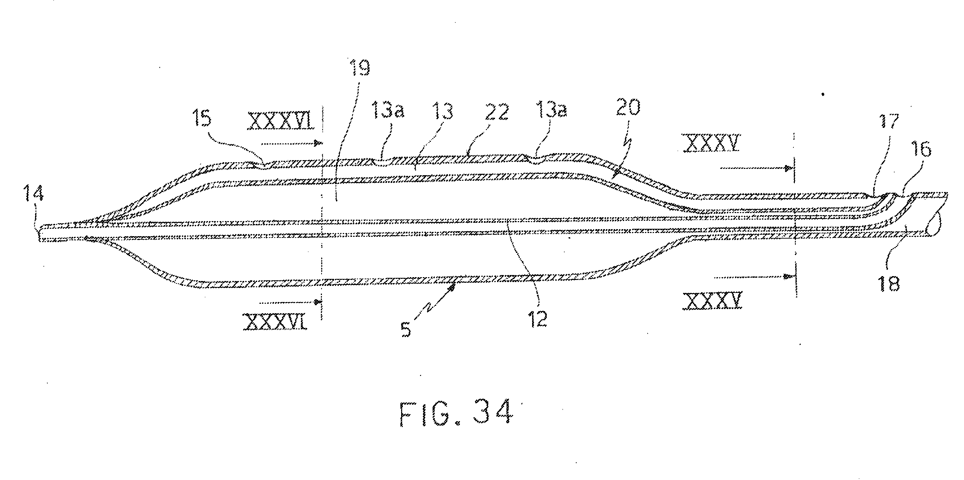

A second guidewire lumen 13, also extending inside the elongated body 2, remains attached to the wall of the expansion means 5, preferably from a distal port 15 to a proximal port 17 (Figures 34, 35). In one possible embodiment, the second guidewire lumen 13 remains attached to the expansion means 5 for a portion of its length, for example comparable to the length of the expansion means 5, from the distal port 15. A second portion of the second guidewire lumen 13 approaching the proximal port 17 remains inside the device but separate from the wall of the actual body and independent of the first guidewire lumen 12 (Figures 32, 33).

The distal ports 14, 15 and proximal ports 16, 17 allow guidewires 24, 25 to fit into said first and second lumens 12, 13 (Figure 19).

The distal ports 14, 15 are preferably spaced out along the elongated body 2. For example, the distal port 14 of the first guidewire lumen 12 is provided at the distal end of the apical portion 9, and the distal port 15 of the second guidewire lumen 13 is provided near the distal end of the expansion means 5 (Figures 1-3, 6a, 6b, 18-23). The proximal ports 16, 17 are preferably positioned in the portion of the elongated body 2 that lies between its proximal end and the expansion means 5. For example, said ports 16, 17 are located at a distance ranging between 90 cm and 130 cm, and preferably between 105 cm and 115 cm, from the proximal end, or from the connector means 10 (Figure 1).

According to the invention, the second guidewire lumen 13 has at least one additional port 13a between the distal port 15 and the proximal port 17. For instance, the embodiment seen in Figure 34 has two additional ports 13a. One possible embodiment may have a plurality of additional ports 13a intermediate between the proximal port 17 and the distal port 15, preferably arranged so that the distance between two consecutive ports is constant.

According to the invention, said endolumenal device is a balloon catheter for angioplasty, 1. Said balloon catheter 1 comprises a tubular catheter 2, a proximal connector 10, and an inflatable balloon 5.

The catheter body 2 is tubular. The proximal portion 4 of said tubular body 2 is designed to support and push the distal portion 3. Therefore said proximal portion 4 is less flexible than the distal portion, which must be flexible in order to be able to enter the peripheral branches of a vessel. For example, said proximal portion 4 is made of a biocompatible material, such as biomedical steel or nylon^{™}. Moreover, said proximal portion 4 is designed to be received in a guide catheter (not shown and known per se) which is necessary for maintaining accessibility of the lumen of the vessel on which it is necessary to operate even when the endolumenal device 1 is withdrawn. Said guide catheter is also necessary for introducing, for example, a radio-opaque contrast medium into the vessel. The proximal portion 4 of the catheter body, 2 includes an inflation lumen 18 (Figures 3, 4 and 4a, 6b). Said lumen 18 extends from the proximal end of the catheter body 2 to the inflatable balloon 5.

The proximal connector 10, for example a connector commonly known as a "Luer", is provided at the proximal end of said portion 4 and forms the abovementioned means of connection of the endolumenal device 1 to the device for the controlled activation of the balloon 5. For example, said connector connects the inflation lumen 18 of the balloon 5 to a pressurized fluid source.

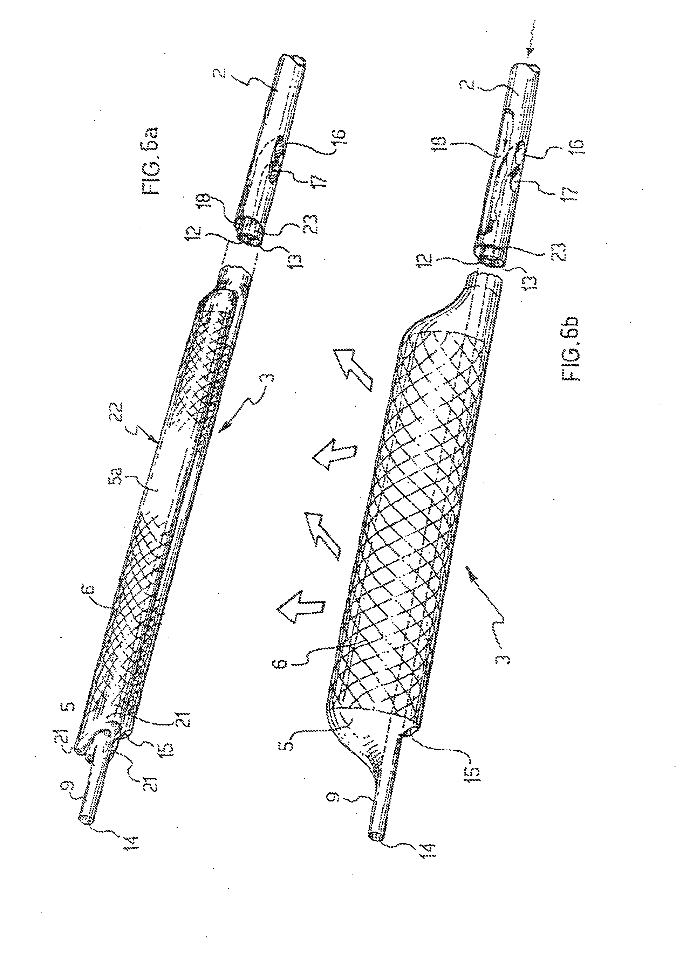

The balloon 5 is associated with the distal portion 3 of the catheter body 2 to form an inflation chamber 19 which at least partially surrounds the catheter body (Figure 3). The inflation chamber 19 is delimited by a balloon wall 20 equipped with an external envelope 22. Said inflation chamber 19 is in communication with the inflation lumen 18. In one embodiment, the balloon includes, between a distal portion 7 and a proximal portion 8, a central portion 5a. Said central portion 5a, when it is in a radially expanded, or inflated, position, is roughly cylindrical. The balloon wall 20 in one embodiment is non-extendable or rigid when subjected to pressurized fluid. Therefore the balloon wall 20, when it is in a radially collapsed position, is folded around the catheter body 2, for example it is threefolded or, in other words, forms three folds 21 (Figure 6a). By means of the external envelope 22, the balloon wall 20 can be removably fitted with an endolumenal prosthesis. For example, the external envelope is removably fitted with an endovascular stent of metallic tubular mesh, plastically deformable from a radially collapsed condition to a radially expanded condition, which can be fixed by pressure to the internal surface of a vessel wall. For this reason, the diameter of the central cylindrical portion 5a, when the balloon is radially expanded or inflated by pressurized fluid injected through the inflation lumen 18, is such as to fix said prosthesis to the wall of the vessel by pressure (Figure 6b).

A longitudinal portion of the balloon wall 20 can be associated internally with the catheter body 2. In other words, said wall 20 is fixed along its entire length to the catheter body, so that when the balloon 5 changes from the radially collapsed or deflated position to the radially expanded or inflated position, said balloon 5 will extend preferably eccentrically or asymmetrically with respect to the catheter body 2, or in other words, on only one side of the body (Figures 3, 5 and 6b).

The distal portion 7 and the proximal portion 8 of the balloon 5 are advantageously pointed in shape. In particular, said portions are frustoconical according to the invention.

Advantageously, the tubular catheter body 2 includes sheath means or sleeve means 23, for example a flexible conduit. For example, said sheath means are an integral part of the elongated body. The sheath means 23 include a tubular body through which run a number of longitudinal lumens, 12, 13 forming the abovementioned guidewire lumens. The guidewire lumens 13, 14, or sections of these, advantageously run in parallel along the elongated body. Said lumens debouch at the ends of the sheath means with the abovementioned guidewire ports 14, 15, 16, 17. Said sheath means 23 are located inside the tubular catheter body 2 in such a way as to leave a space (which forms the abovementioned inflation lumen 18) along the entire length of that portion of the catheter body 2 which is situated between the proximal connector 10 and the balloon 5. Preferably, said sheath means are attached for their entire length to the portion of the wall of the catheter body opposite the inflation chamber 19 (Figures 3, 4, 4a and 6b). The ends of the sheath means 23 are attached to the wall of the catheter body in such a way as to make the guidewire lumens accessible from outside the catheter body through the guidewire ports.

It is particularly advantageous when said sheath means 23 are attached to the catheter body so that they debouch in a first distal guidewire port 14 of the first guidewire lumen 12 distant from a second distal guidewire port 15 of the second guidewire lumen 13.

In particular, said sheath means extend to the tip of the distal portion 3 of the catheter body 2 in such a way as to debouch with the first distal guidewire port to the tip of the apical tract 9.

In one possible embodiment (Figures 32 and 34), the sheath means 23 comprise a tubular body pierced longitudinally by the first lumen 12 forming the guidewire lumen described above. This sheath extends through the elongated body 2 from the proximal end portion 4 to the distal end portion 3. The second guidewire lumen 13, or at least a section of the latter, advantageously runs through the elongated body 2 attached to the expansion means 5, that is in the balloon wall 20. The wall part 20 that houses the second guidewire lumen 13 is preferably the balloon part that can expand asymmetrically with respect to the elongated body 2. In one possible embodiment, the guidewire lumen 13 runs inside the balloon wall 20 from its proximal port 17 to its distal port 15 (Figure 34). In a different embodiment, only a portion of the second guidewire lumen 13 runs inside the balloon wall 20, preferably the portion corresponding to the extension of the expansion means 5, that is extending from the distal port 15 to an intermediate point of the wall. From this intermediate point and as far as the proximal port 17, the second lumen 13 may run independently inside the elongated body 2 defined by other sheath means 23a (Figure 32).

The first lumen 12 has an open end at one end of the sheath means with the above-described guidewire ports 14 and 16. The lumen 13 ends at the end of the balloon wall 20 with the above-described guidewire ports 15 and 17 and, if desired, with the additional one or more ports 13a. Said sheath means 23, and, if desired, the additional sheath means 23a, are located inside the tubular catheter body 2 in such a way as to leave a space, forming the abovementioned inflation lumen 18, along the full length of the catheter body 2 situated between the proximal connector 10 and the balloon 5.

For example thanks to the asymmetrical position of the balloon 5 with respect to the catheter body 2, the second distal guidewire port 15 is positioned along the catheter body 2 so as to allow the second guidewire lumen 13 to debouch at the distal end of the central portion 5a of the balloon, or in other words, so as to be positioned just outside the prosthesis 6 attachable to the balloon 5 (Figures 1 to 6b).

In a further embodiment of the invention, the second distal guidewire port 15 is positioned along the catheter body in such a way that the second guidewire lumen 13 debouches at a point located between the distal portion 7 and the proximal portion 8 of the balloon 5, and in particular at a point of the wall opposite the central portion 5a attachable to the prosthesis 6. For example, said port 15 is located near the centre line of said central portion, 5a. Preferably, the prosthesis 6, which can be attached to said catheter 1, has a window 26 designed to prevent obstruction of said distal guidewire port 15 when it is fitted on the balloon, 5. For example, the prosthesis 6 has a wider mesh 26 than the other meshes of the prosthesis, and at the same time of a size close to that of the ostium of access to the lumen of the branch on which it is necessary to operate, or only slightly smaller. Alternatively, the balloon can be fitted with a number of prostheses, placed side by side in order to avoid obstructing said port 15.

Preferably, the proximal guidewire ports 16, 17 are located in a portion of the catheter body 2 which, during use of the catheter 1, remains sheathed in the guide catheter. Alternatively, said ports 16, 17 are located at the proximal end of the catheter body. In this case the balloon catheter 1 is fitted with a proximal connector 10 with at least two channels. A first channel for the admission of the pressurized fluid into the inflation lumen 18, and a second channel for passing the guidewires 24, 25 along. For example, said proximal guidewire ports are located at a distance from the tip of the catheter ranging between 15 cm and 35 cm, and preferably between 20 cm and 30 cm.

Advantageously, radio-opaque markers 30 and 31 are associated with the catheter body 2 (Figure 3). For example, said markers are located along the catheter body 2 at the distal and proximal ends of the prosthesis 6.

Said catheter body also includes radio-opaque markers for the identification of the position along said body of the distal 14, 15, and/or proximal 16, 17 guidewire ports of the guidewire lumens 13, 14.

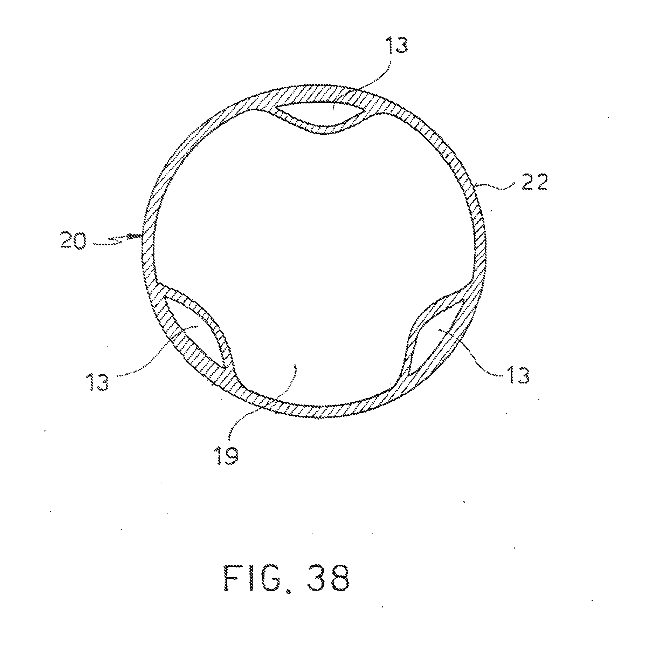

In another embodiment, both the guidewire lumens 12 and 13, or at least a section of these, advantageously run in the wall of the elongated body 2. This section preferably corresponds to the longitudinal section of the expansion means 5. In another embodiment, there are more than two guidewire lumens in the balloon wall 20, preferably three arranged at 120° from each other (Figure 38). In this latter embodiment, it is possible to avoid the guidewire lumen 12 positioned inside the elongated body 2, as described earlier.

A kit for delivering and positioning an endolumenal expandable prosthesis may comprise an endolumenal device, 1, as described above, at least one pair of guidewires 24, 25, and at least one expandable prosthesis 6 radially associated with the expansion means 5 of said endolumenal device 1. Said prosthesis comprises a tubular prosthesis body adaptable from a radially collapsed condition, of minimal external diameter, to a radially expanded condition, of extended external diameter greater than the collapsed external diameter.

For example, said kit for delivering and positioning an endolumenal expandable prosthesis comprises at least one first radially expandable prosthesis associated with the proximal portion of the expansion means of said endolumenal device and also comprises at least one second radially expandable prosthesis associated with the distal portion of the expansion means of said endolumenal device, or alternatively a single prosthesis overlapping said proximal and distal portions of the expansion means.

Each of the guidewires of said kit includes means of identification, such as for example the colour of at least a proximal portion of the guidewire, or a diameter of the cross section of a proximal portion of the guidewire which differs for each guidewire.

Said guidewires advantageously comprise an elastically flexible distal end portion.

In particular, said guidewires include initial proximal sections which are positionable along a proximal section of path common to all the guidewires, and secondary distal sections which are positionable along distal sections of path which diverge and form with said proximal section of path a bifurcation. It is particularly advantageous for at least one of said guidewires to include an elastically flexible distal portion which extends at least to straddle said bifurcation.

It is furthermore advantageous for said guidewires to include radio-opaque markers, for example located at the tip of the distal portion.

A description of the working of an endolumenal device according to this invention follows.

In particular, the operations necessary for guiding an endolumenal device along guidewires 24, 25 are described below. Said guidewires are located along a common proximal section of path and a diverging distal section of path, forming a bifurcation between said sections. The above method comprises the following stages:
- said endolumenal device is fitted onto a proximal end of a first guidewire so that said first guidewire is received in a first guidewire lumen through its distal guidewire port;
- said endolumenal device is fitted onto a proximal end of a second guidewire so that said second guidewire is received in a second guidewire lumen through its distal guidewire port;
- said endolumenal device is advanced along said guidewires until at least part of the distal end portion of the elongated body is positioned beyond the bifurcation of the guidewires.

Advantageously, it is possible to envisage a further method of guiding an endolumenal device along guidewires 24, 25, in which said guidewires are positioned along a common proximal section of path and a diverging, distal section of path, forming between said sections a bifurcation. This further method includes the following stages:
- said endolumenal device is fitted onto a proximal end of a first guidewire so that said first guidewire is received in a first guidewire lumen through its distal guidewire port;
- said endolumenal device is fitted onto a proximal end of a second guidewire so that said second guidewire is received in a second guidewire lumen through its distal guidewire port;
- said endolumenal device is advanced along said guidewires until at least part of the distal end portion of the elongated body lies on a distal divergent section of path of one of the guidewires.

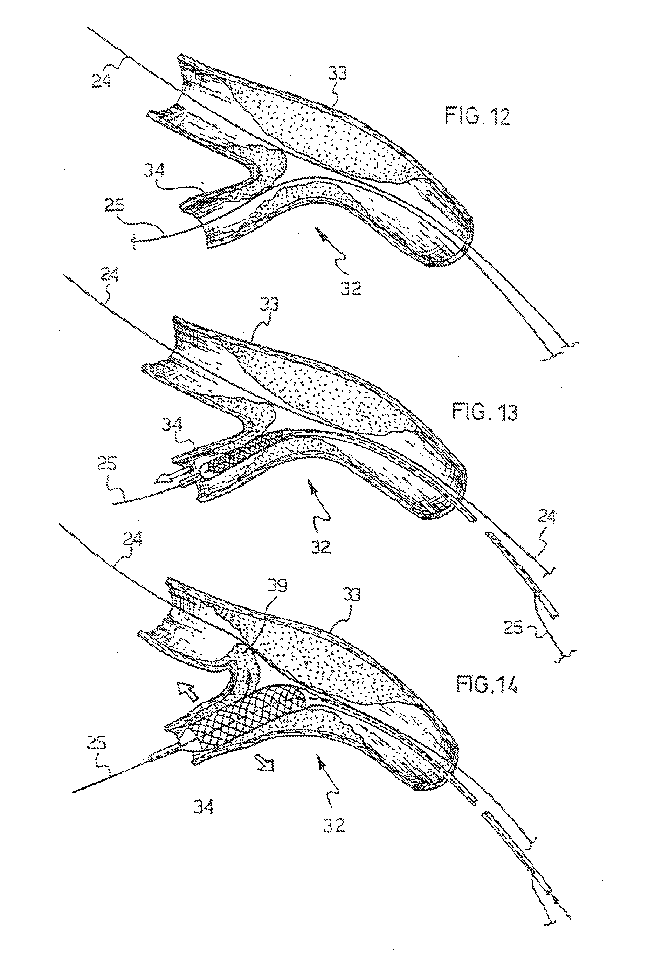

The steps of a method for fitting radially expandable prostheses to the walls of branches forming a 'T bifurcation' 32 are described below (Figures 12 to 17e). Said bifurcation 32 comprises a principal conduit 33 and a secondary conduit 34 that branches off from a wall of the principal conduit 33. The abovementioned method comprises the following steps:

A kit as described above, and in particular a kit which comprises an endolumenal device having a distal guidewire port located inside a central portion of the expansion means, is prepared.

Then, through a proximal section of the principal conduit, a first guidewire is positioned in the principal conduit so that it passes the bifurcation, and a second guidewire is positioned in the secondary conduit. Said guidewires are positioned in such a way as to follow an initial proximal section of path together and second distal sections of path that diverge at said bifurcation (Figure 12).

Next, a first endolumenal device equipped with a radially expandable prosthesis is fitted onto a distal end of the second guidewire, so that said second guidewire is received in a guidewire lumen of the endolumenal device through a distal guidewire port located on the tip of its elongated body.

Said first endolumenal device is inserted into said conduits following the proximal and then the distal sections of path of the second guidewire in order to position the radially expandable prosthesis in the secondary conduit so that its proximal edge is positioned near a mouth of said secondary conduit (Figure 13).

Said expandable means are then activated so that said prosthesis is in its radially expanded condition and fixed by pressure to the wall of the secondary conduit (Figure 14).

Next, said expansion means are withdrawn and the first endolumenal device is withdrawn from the second guidewire until it has been removed from the conduits.

A second endolumenal device equipped with a radially expandable prosthesis is fitted onto a proximal end of the first guidewire so that said first guidewire is received in a first guidewire lumen through its distal guidewire port located on the tip of the endolumenal device, and said second endolumenal device is fitted onto a proximal end of the second guidewire so that said second guidewire is received in a second guidewire lumen through its distal guidewire port located on the portion of elongated body that lies between a distal and a proximal end of the expansion means.

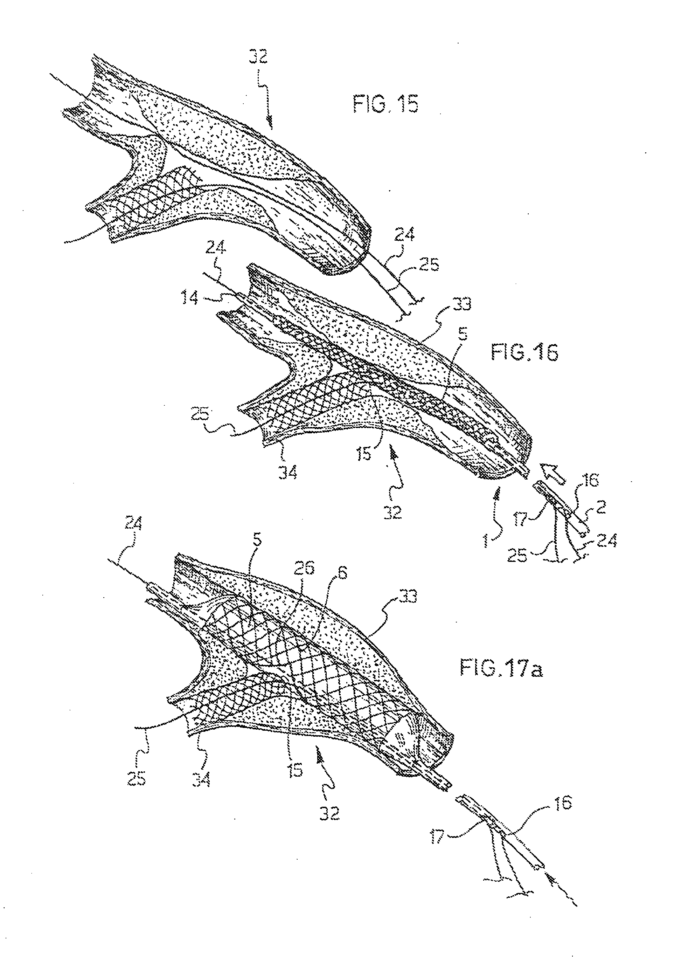

Said endolumenal device is inserted into the principal conduit and slid along the proximal section of path of the guidewires until a distal portion of the endolumenal device, located between the tip of said device and the distal guidewire port of the second guidewire lumen, is positioned beyond the bifurcation (Figure 16).

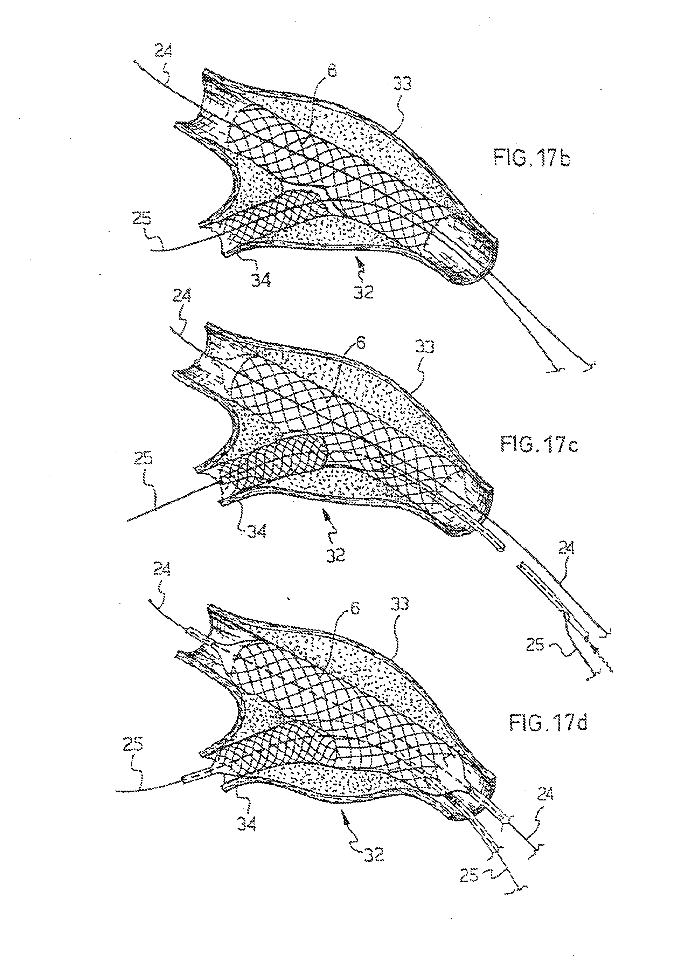

The expandable means of said second device are activated so as to bring said prosthesis into its radially expanded condition and fixed by pressure to the wall of the primary conduit and straddling the bifurcation (Figure 17a).

Finally said expansion means are withdrawn and then the second endolumenal device is withdrawn from the guidewires until it has been removed from the conduits.

Further steps which make it possible to adapt the previously implanted prostheses in order to cover the lesion completely are described below.

A third endolumenal device without a prosthesis is fitted onto the second guidewire, positioning it to straddle the bifurcation so that a distal portion of the expansion means enters the secondary conduit and a proximal portion of the expansion means is positioned in the principal conduit.

The expansion means of the third device are then activated so as to adapt a portion of the prosthesis in the principal conduit facing the mouth or lateral window of the secondary conduit to the shape of the lumen of said secondary conduit (Figure 17c).

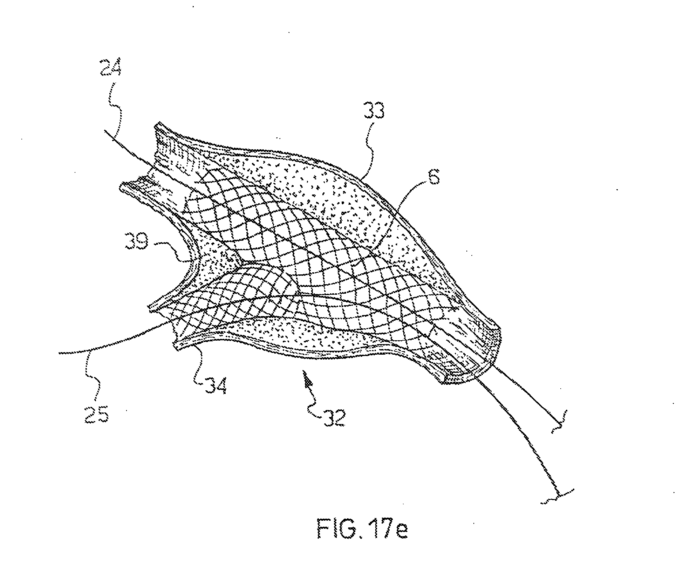

Said expansion means are withdrawn and then the third endolumenal device is withdrawn from the second guidewire until it has been removed from the conduits (Figure 17e).

By inflating the third endolumenal device (for example a balloon catheter for angioplasty) straddling the bifurcation, the mesh of the prosthesis implanted in the principal conduit is moulded so that it surrounds the ostium of the secondary conduit perfectly, and guarantees perfect coverage of the damaged area (Figure 17c). Alternatively, particularly in the case of larger diameter or larger bore conduits it is possible to insert two balloon catheters simultaneously, fitting them on the guidewires 24, 25, so that they are paired and straddle the bifurcation, one in the principal conduit and the second partially in the secondary conduit and partially in the principal conduit. Simultaneous expansion of the two balloons both shapes the prostheses so that they match and form a continuous support structure which covers the entire extension of the lesion and creates, in the area of the bifurcation, a funnel-shaped area which joins the principal and the secondary branches and promotes non-vortical circulation of fluid in the conduits or vessels.

The stages of the method described above may also be reversed, implanting first the principal vessel and then the secondary vessel.

In view of the above procedures it is evident that the implanting of a prosthesis in the principal vessel causes the plaque 39 to obstruct the ostium of the secondary vessel or vice versa. Thanks to the fact that, using the device according to the invention, the application of a first prosthesis in a vessel is always carried out leaving a second guidewire in a second branch, in spite of the presence in the mouth of the same of a wall of plaque caused by snow-plow or plaque-shifting. It is therefore always possible to insert in the second branch a device for the application of a second prosthesis. Using known prior-art devices it is not possible to operate simultaneously with two guidewires always present in the two branches of the bifurcation because a second guidewire not positioned inside the prior-art device would be externally walled by the prosthesis and rendered unusable. In other words, with the prior-art device it is necessary to proceed using only one guidewire. With the device according to the invention, however, it is possible to effect the swift exchange of the endolumenal device on guidewires which remain in situ, it being possible to withdraw the endolumenal device from a first branch of the bifurcation to reinsert the same device or a second device in a second branch with extreme rapidity.

The steps for a further method for fitting radially expandable prostheses to the walls of the branches of conduits forming a 'Y bifurcation' 35 are described below. Said bifurcation comprises a proximal principal conduit 36 and a first and a second secondary distal conduits 37, 38 which branch off from a distal end of the principal conduit, forming between them a carina. Said method comprises the following steps.

A kit as described above is prepared, and in particular a kit comprising an endolumenal device fitted with a distal guidewire port located near the distal edge of a prosthesis fitted on the expansion means, and a second distal guidewire port located at the tip of the device, or apical port.

Through the principal conduit a first guidewire is positioned in the first secondary conduit and a second guidewire in the second secondary conduit, said guidewires being positioned so as to follow a first proximal section of path together and second distal section of path that diverge after said bifurcation (Figure 18).

A first endolumenal device equipped with a radially expandable prosthesis is fitted onto a proximal end of the first guidewire, so that said first guidewire is received in a guidewire lumen of the endolumenal device through its distal guidewire port located at the tip of its elongated body.

Said first endolumenal device is fitted onto a proximal end of the second guidewire so that said second guidewire is received in a second guidewire lumen through its distal guidewire port located near the stent distal edge, just beyond the prosthesis.

Said first endolumenal device is inserted into said conduits following the proximal section of path until the carina is positioned against the elongated body and near the distal guidewire port positioned near the distal end of the expansion means (Figure 19).

Said expandable means are activated so as to bring said prosthesis into its radially expanded condition, fixed by pressure to the wall of the principal conduit (Figure 20).

Said expansion means are withdrawn and the first endolumenal device is then withdrawn from the guidewires.

A second endolumenal device equipped with a radially expandable prosthesis is fitted onto a proximal end of the first guidewire so that said guidewire is received in a guidewire lumen through its distal guidewire port located on the tip of said second endolumenal device.

A third endolumenal device equipped with a radially expandable prosthesis is fitted, at the same time as the second endolumenal device, onto a proximal end of the second guidewire so that said second guidewire is received in a guidewire lumen through its distal guidewire port located on the tip of said third endolumenal device.

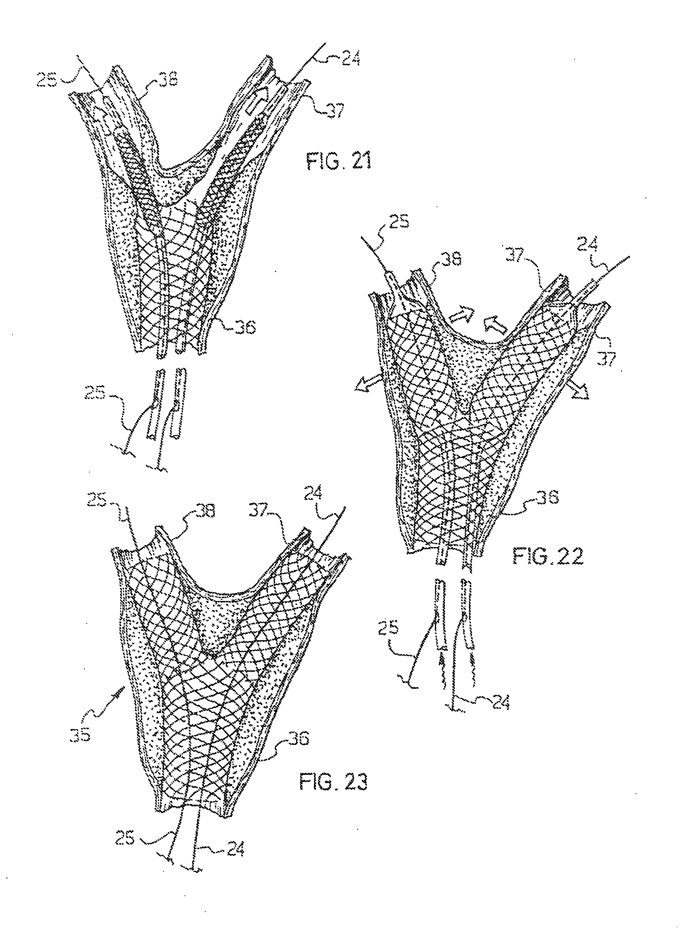

Said second and third endolumenal devices are simultaneously inserted into the principal conduit and slid along the proximal section of path of the guidewires and then along the respective distal sections of path of said guidewires, until the expansion means are positioned in a proximal portion of said first and second secondary conduits, so that a proximal edge of the expansion means is positioned near the carina. In particular, care is taken to ensure that the proximal edge of both the second and third prostheses is in contact with the distal edge of the first prosthesis, already positioned and expanded in the principal lumen (Figure 21).

The expansion means of said second and third endolumenal devices are activated in order to bring the respective prostheses into a radially expanded condition fixed by pressure to the walls of said first and second conduits (Figure 22).

Said expansion means are withdrawn and then the second and third endolumenal devices are withdrdawn from the guidewires until they have been removed from the conduits (Figure 23).

The above description shows how the use of at least two guidewire lumens which extend at least partially along the inside of the elongated body makes it possible to fit the endolumenal device simultaneously on at least two guidewires. In this manner, once at least two guidewires have been inserted in the branches of a bifurcation, it will be possible to insert and withdraw the endolumenal device from a first branch of the bifurcation without ever losing rapid access to all the branches already negotiated, i.e. reached by guidewires. In other words, it will be possible to maintain uninterrupted access or vascular approach to all the branches of the vascular system on which it is necessary to operate and in which a guidewire has been inserted or, in yet other words, using the device proposed it is no longer necessary to break through the wall of plaque 39 which obstructs the ostium of the branch by snow-plow or plaque-shifting.

Thanks to the endolumenal device according to the invention it will also be possible to position accurately a first endovascular prosthesis in the principal vessel always with precise positioning and complete distension or application of the prosthesis over the entire area of the lesion, thus reducing the probability of re-stenosis and avoiding the pitfalls of the known techniques.

Advantageously, the endolumenal device proposed allows extreme flexibility and modularity in the application of the endolumenal prostheses. Thus, if the expansion means are positioned exactly straddling the bifurcation it is possible to implant endolumenal prostheses of exactly the correct length and diameter for the dimensions of the segment of damaged vessel to be treated, by means of the proximal and distal portions of the expansion means.

With further advantage, each portion of the expansion means makes it possible to implant a number of endolumenal prostheses of optimal diameter and length for the anatomy of the damaged vascular branch.

When expansion means fitted to the endolumenal prostheses are in the collapsed position, the device according to the invention is of reduced transverse bulk, making it possible to reach peripheral branches extremely easily and rapidly (trackability).

Together with the versatility of application of prostheses adapted to different branches of the bifurcation, the device proposed also makes it possible to join prostheses, or, in other words, it allows total coverage of the damaged area, avoiding prolapse of atheromatous material and reducing the probability of re-stenosis.

A further advantage derives from the fact that, using the endolumenal device according to the invention, the geometry of the prosthesis is not distorted and the vascular anatomy is respected. In contrast, distortion of the prosthesis is inevitable when endolumenal devices according to the prior art are used.

Obviously, variations and/or additions to what is described above and illustrated may be envisaged.

Alternatively to a balloon with rigid walls threefolded onto the catheter body for insertion into the lumen of a vessel, as described above, it is possible to envisage the use of a compliant or extensible balloon.

Other possible variations are:
- the catheter of the type described above, single operator exchange or monorail, may alternatively be of the over the wire type, that is with opening of the proximal guidewire lumens at the proximal end of the elongated body;
- one of the at least two guidewire lumens may always be occupied by a guidewire and may be inserted in the conduit, or vessel, together with the endolumenal device. Preferably, in this case, the guidewire is fastened to or is an integral part of the elongated body of the endolumenal device, for example extending from the apical portion of this (fixed wire).
- the catheter may also be of the perfusion balloon type in which passages are provided for fluid flow when the balloon is inflated: these provide communication between the portions of elongated body above and below the expansion means (passages for the blood in the body to prevent temporary occlusion of the vessel during the application of the prosthesis and the inflation of the balloon).
- the endovascular prosthesis may be modular. For example it is possible to provide a series of prostheses of set diameters and a series of set lengths which the operator can attach to the proximal and distal portions of the expansion means, making them extremely flexible or, in other words, making it possible to adapt the prosthesis perfectly to the pathological requirements of the moment, or in other words, to the size of the lesion and the bore of the lumen of the vessel on which it is necessary to operate.

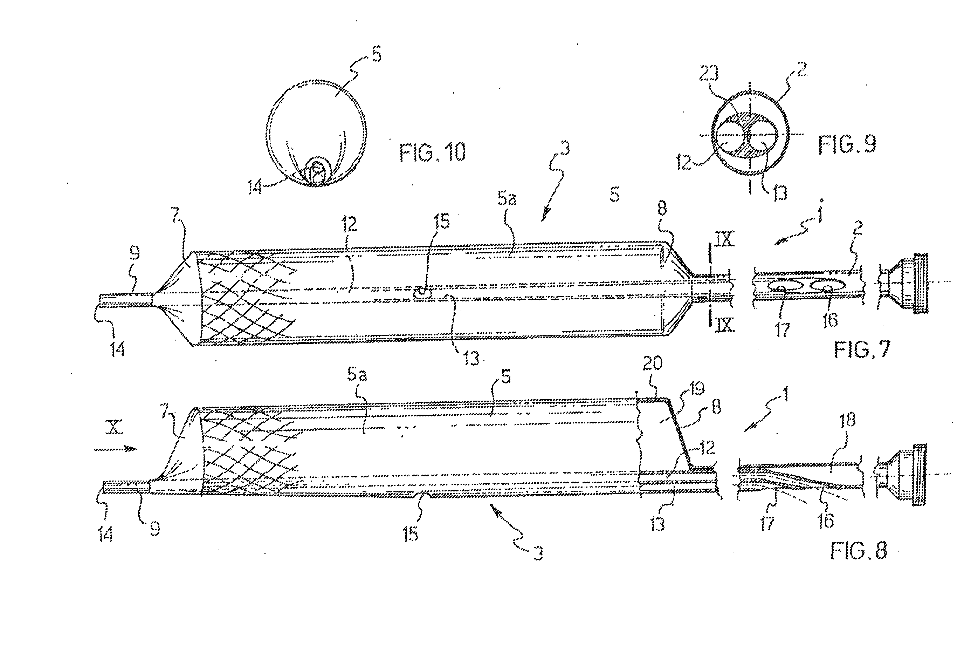

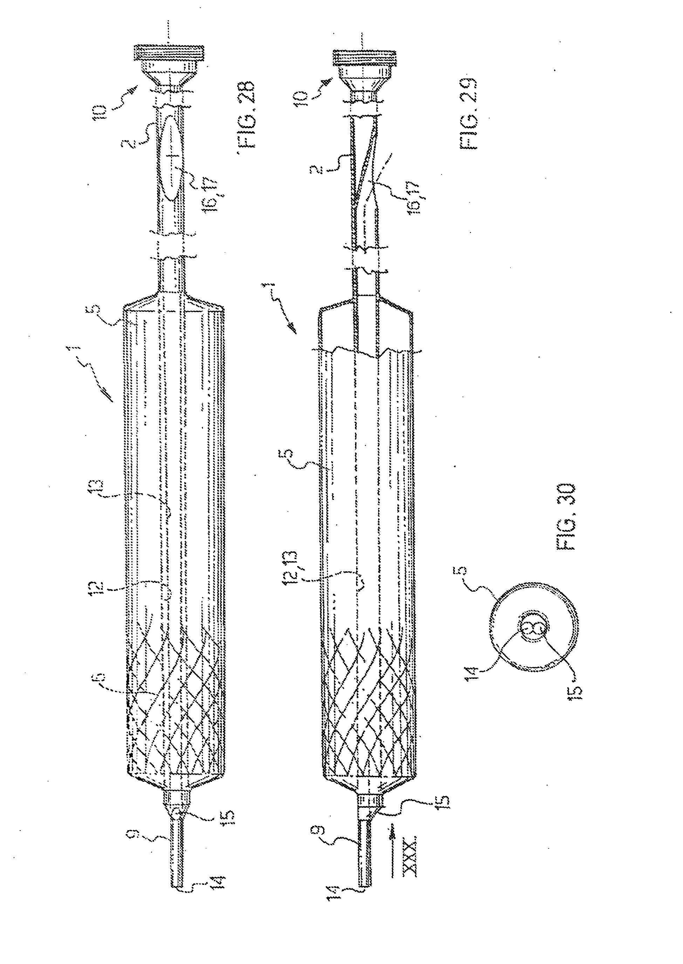

As an alternative to the above description, illustrated by Figures 3 and 8, at least one portion of said at least one pair of guidewire lumens 12, 13 forms a single guidewire lumen (Figures 28, 29 and 30).

Advantageously, the distal guidewire port 15 of a second guidewire lumen 13 is located near a proximal end of the expansion means 5 (Figures 31, 32). A conduit 27, for example a flexible conduit, preferably longitudinally elastic, is functionally connected through its proximal port 28 to said distal guidewire port 15. Said conduit 27 has a distal guidewire port 15a and, being attached along its entire length to the external surface of the expansion means 5, said distal port 15a is located in the central portion 5a of said expansion means 5. In this case, advantageously, the expansion means, for example a balloon 5, expand symmetrically to the elongated body 2.

In a further example, said expansion means are designed to hold a self-expanding prosthesis in a radially folded position and release it in a controlled manner so that it takes up a radially expanded position. Said expansion means include a sheath designed to receive in a sheath lumen said self-expanding prosthesis. Said sheath can advantageously be adapted in controlled manner from a first constricted position in which the self-expanding prosthesis is confined in said lumen of the sheath, to a second released position, in which said prosthesis is released from said lumen of the sheath so that said prosthesis is radially free, to bring itself into the radially expanded condition.

Such a device can be advantageously used in the artificial conduits of biomedical equipment that connects up to the patient's body. For example, a device of the type described above can be used for delivering, positioning and installing an element for the repair of the walls of a conduit accidentally damaged during the use of the abovementioned machinery.

A person skilled in the art could make numerous changes and adaptations to the preferred embodiment of the endolumenal device described above or substitute elements with others functionally equivalent, in order to meet contingent and specific requirements, without however departing from the scope of the following claims.

## Claims

1. Endolumenal device (1) for delivering and positioning an endolumenal expandable prosthesis (6) for a bifurcation between a principal conduit and at least one secondary conduit, comprising:
- an elongated body (2) having a proximal end portion (4) and a distal end portion (3);
- the distal end portion (3) of said elongated body (2) comprising expansion means (5) having a longitudinally extended active portion removably engageable with the endolumenal expandable prosthesis (6) and adapted to adjust said prosthesis (6) from a radially collapsed condition to a radially expanded condition;
- a guidewire tracking means (11) extending at least partially along said elongated body (2);
in which
- said guidewire tracking means (11) comprises at least one pair of guidewire lumens (12, 13), at least one of said guidewire lumens (13) extending at least partially remaining attached to the wall of said expansion means (5) and having at least one distal port (15) on a side opposite the expansion means and adapted to receive through said port a portion of at least one guidewire positionable with its distal portion in said principal conduit or in said at least one secondary conduit,
- said at least one of said guidewire lumens (13) that extends at least partially remaining attached to the wall of said expansion means (5) comprises a proximal port (17), a distal port (15) and at least one intermediate additional port (13a) between said distal port and said proximal port,
- said endolumenal device is a balloon catheter for angioplasty (1) comprising a tubular catheter (2), a proximal connector (10), and an inflatable balloon (5),
- said guidewire lumens run inside the balloon wall (20) from proximal ports to the distal ports (15),
said proximal ports (16, 17) are positioned in the portion of the elongated body (2) that lies between its proximal end and the expansion means (5),
**characterized in that**
- said balloon (5) has a distal portion (7) and a proximal portion (8), said distal (7) and proximal (8) portions of the balloon (5) are frustoconical shanks.

2. Endolumenal device (1), according to Claim 1, in which said lumens attached to the wall are two or three in number.

3. Endolumenal device (1), according to Claim 1 or 2, in which said guidewire lumens (12, 13) have distal guidewire ports (14, 15) spaced out along said elongated body (2).

4. Endolumenal device (1), according to Claim 3, in which a first guidewire lumen (12) has said distal guidewire port (14) at the free end or tip of said distal end portion (3) of the elongated body (2) and said guidewire tracking means (11) comprise at least one of said guidewire lumens in an approximately central position with respect to said expansion means considered in cross section at right angles to the endolumenal device.

5. Endolumenal device (1), according to Claim 3 or 4, in which a second guidewire lumen (13) has said distal guidewire port (15) near a distal end of the expansion means (5).

6. Endolumenal device (1), according to Claim 3 or 4, in which a second guidewire lumen (13) has said distal port (15) in a portion of the elongated body (2) that lies between a distal end and a proximal end of the expansion means (5).

7. Endolumenal device (1), according to Claim 6, in which said distal guidewire port (15) of said second guidewire lumen (13) is provided near the centre line of that portion of the elongated body (2) that lies between said distal and proximal ends of the expansion means (5).

8. Endolumenal device (1), according to any of the previous claims, in which said guidewire lumens (12, 13) have proximal guidewire ports (16, 17) located in portions of the elongated body (2) located, with respect to the expansion means (5), at the opposite end from its distal end (9).

9. Endolumenal device (1), according to Claim 8, in which said proximal ports (16, 17) are located at the proximal tip of the proximal portion (4) of the elongated body (2).

10. Endolumenal device (1), according to any of the previous claims, in which portions of the guidewire lumens of said at least one pair of guidewire lumens (12, 13) run in parallel inside the elongated body (2).

11. Endolumenal device (1), according to any of the previous claims, in which at least one portion of said at least one pair of guidewire lumens (12, 13) forms a single guidewire lumen.

12. Endolumenal device (1), according to Claim 3, in which the distal guidewire port (15) of a second guidewire lumen (13) is located near a proximal end of the expansion means (5).

13. Endolumenal device (1), according to Claim 1, in which the guidewire lumens (12, 13) have distal ports (14, 15) located near a proximal end of the expansion means (5).

## Patentansprüche

1. Endoluminale Vorrichtung (1) zum Einbringen und Positionieren einer endoluminalen aufweitbaren Prothese (6) für eine Verzweigung zwischen einer Hauptleitung und wenigstens einer Sekundärleitung, umfassend:
- einen länglichen Körper (2), welcher einen proximalen Endabschnitt (4) und einen distalen Endabschnitt (3) aufweist;
- wobei der distale Endabschnitt (3) des länglichen Körpers (2) Aufweitungsmittel (5) umfasst, welche einen sich longitudinal erstreckenden aktiven Abschnitt aufweisen, welcher entfernbar mit der endoluminalen aufweitbaren Prothese (6) in Eingriff bringbar und dazu eingerichtet ist, die Prothese (6) aus einem radial verengten Zustand in einen radial aufgeweiteten Zustand einzustellen;
- Führungsdraht-Nachführungsmittel (11), welche sich wenigstens teilweise entlang des länglichen Körpers (2) erstrecken; wobei
- die Führungsdraht-Nachführungsmittel (11) wenigstens ein Paar von Führungsdraht-Lumina (12, 13) umfassen, wobei sich wenigstens eines der Führungsdraht-Lumina (13) wenigstens teilweise an der Wand der Aufweitungsmittel (5) angebracht verbleibend erstreckt und wenigstens eine distale Öffnung (15) an einer den Aufweitungsmitteln gegenüberliegenden Seite aufweist und dazu eingerichtet ist, durch die Öffnung einen Abschnitt wenigstens eines Führungsdrahts aufzunehmen, welcher mit dessen distalen Abschnitt in der Hauptleitung oder in der wenigstens einen Sekundärleitung positionierbar ist,
- wobei das wenigstens eine der Führungsdraht-Lumina (13), welches sich wenigstens teilweise an der Wand der Aufweitungsmittel (5) angebracht verbleibend erstreckt, eine proximale Öffnung (17), eine distale Öffnung (15) und wenigstens eine zusätzliche Zwischenöffnung (13a) zwischen der distalen Öffnung und der proximalen Öffnung umfasst,
- wobei die endoluminale Vorrichtung ein Angioplastie-Ballonkatheter (1) ist, welcher einen rohrförmigen Katheter (2), ein proximales Verbindungselement (10) und einen aufblasbaren Ballon (5) umfasst,
- wobei die Führungsdraht-Lumina innerhalb der Ballonwand (20) von proximalen Öffnungen zu den distalen Öffnungen (15) verlaufen, wobei die proximalen Öffnungen (16, 17) in dem Abschnitt des länglichen Körpers (2) angeordnet sind, welcher zwischen dessen proximalen Ende und den Aufweitungsmitteln (5) liegt,
**dadurch gekennzeichnet, dass** der Ballon (5) einen distalen Abschnitt (7) und einen proximalen Abschnitt (8) aufweist, wobei der distale (7) und der proximale (8) Abschnitt des Ballons (5) kegelstumpfförmige Schafte sind.

2. Endoluminale Vorrichtung (1) nach Anspruch 1,
wobei die Anzahl der an der Wand angebrachten Lumina zwei oder drei beträgt.

3. Endoluminale Vorrichtung (1) nach Anspruch 1 oder 2,
wobei die Führungsdraht-Lumina (12, 13) distale Führungsdraht-Öffnungen (14, 15) aufweisen, welche entlang des länglichen Körpers (2) beabstandet sind.

4. Endoluminale Vorrichtung (1) nach Anspruch 3,
wobei ein erstes Führungsdraht-Lumen (12) die distale Führungsdraht-Öffnung (14) an dem freien Ende oder der Spitze des distalen Endabschnitts (3) des länglichen Körpers (2) aufweist und wobei die Führungsdraht-Nachführungsmittel (11) wenigstens eines der Führungsdraht-Lumina in einer näherungsweise mittleren Position in Bezug zu einem rechtwinklig zu der endoluminalen Vorrichtung verlaufenden Querschnitt der Aufweitungsmittel umfassen.

5. Endoluminale Vorrichtung (1) nach Anspruch 3 oder 4,
wobei ein zweites Führungsdraht-Lumen (13) die distale Führungsdraht-Öffnung (15) in der Nähe eines distalen Endes der Aufweitungsmittel (5) aufweist.

6. Endoluminale Vorrichtung (1) nach Anspruch 3 oder 4,
wobei ein zweites Führungsdraht-Lumen (13) die distale Öffnung (15) in einem Abschnitt des länglichen Körpers (2) aufweist, welcher zwischen einem distalen Ende und einem proximalen Ende der Aufweitungsmittel (5) liegt.

7. Endoluminale Vorrichtung (1) nach Anspruch 6,
wobei die distale Führungsdraht-Öffnung (15) des zweiten Führungsdraht-Lumens (13) in der Nähe der Mittenlinie desjenigen Abschnitts des länglichen Körpers (2) bereitgestellt ist, welcher zwischen dem distalen und dem proximalen Ende der Aufweitungsmittel (5) liegt.

8. Endoluminale Vorrichtung (1) nach einem der vorhergehenden Ansprüche,
wobei die Führungsdraht-Lumina (12, 13) proximale Führungsdraht-Öffnungen (16, 17) aufweisen, welche sich in Abschnitten des länglichen Körpers (2) befinden, welche sich in Bezug zu den Aufweitungsmitteln (5) an dem gegenüberliegenden Ende von dessen distalen Ende (9) befinden.

9. Endoluminale Vorrichtung (1) nach Anspruch 8,
wobei sich die proximalen Öffnungen (16, 17) an der proximalen Spitze des proximalen Abschnitts (4) des länglichen Körpers (2) befinden.

10. Endoluminale Vorrichtung (1) nach einem der vorhergehenden Ansprüche,
wobei Abschnitte der Führungsdraht-Lumina des wenigstens einen Paars von Führungsdraht-Lumina (12, 13) parallel innerhalb des länglichen Körpers (2) verlaufen.

11. Endoluminale Vorrichtung (1) nach einem der vorhergehenden Ansprüche,
wobei wenigstens ein Abschnitt des wenigstens einen Paars von Führungsdraht-Lumina (12, 13) ein einzelnes Führungsdraht-Lumen bildet.

12. Endoluminale Vorrichtung (1) nach Anspruch 3,
wobei sich die distale Führungsdraht-Öffnung (15) eines zweiten Führungsdraht-Lumens (13) in der Nähe eines proximalen Endes der Aufweitungsmittel (5) befindet.

13. Endoluminale Vorrichtung (1) nach Anspruch 1,
wobei die Führungsdraht-Lumina (12, 13) distale Öffnungen (14, 15) aufweisen, welche sich in der Nähe eines proximalen Endes der Aufweitungsmittel (5) befinden.

## Revendications

1. Dispositif endoluminal (1) pour délivrer et positionner une prothèse endoluminale expansible (6) pour une bifurcation entre un conduit principal et au moins un conduit secondaire, comprenant:
un corps allongé (2) ayant une partie d'extrémité proximale (4) et une partie d'extrémité distale (3);
la partie d'extrémité distale (3) dudit corps allongé (2) comprenant des moyens d'expansion (5) ayant une partie active longitudinalement étendue pouvant se mettre en prise de manière amovible avec la prothèse endoluminale expansible (6) et adaptée pour ajuster ladite prothèse (6) d'une condition radialement repliée à une condition radialement expansée;
des moyens suiveurs de fil guide (11) s'étendant au moins partiellement le long dudit corps allongé (2);
dans lequel:
lesdits moyens suiveurs de fil guide (11) comprennent au moins une paire de lumières de fil guide (12, 13), au moins l'une desdites lumières de fil guide (13) s'étendant au moins partiellement en restant fixée à la paroi desdits moyens d'expansion (5) et ayant au moins un orifice distal (15) sur un côté opposé aux moyens d'expansion et adaptée pour recevoir à travers ledit orifice, une partie du au moins un fil guide positionnable avec sa partie distale dans ledit conduit principal ou dans ledit au moins un conduit secondaire,
ladite au moins une desdites lumières de fil guide (13) qui s'étend au moins partiellement en restant fixée à la paroi desdits moyens d'expansion (5) comprend un orifice proximal (17), un orifice distal (15) et au moins un orifice supplémentaire intermédiaire (13a) entre ledit orifice distal et ledit orifice proximal,
ledit dispositif endoluminal est un cathéter à ballonnet pour angioplastie (1) comprenant un cathéter tubulaire (2), un connecteur proximal (10) et un ballonnet gonflable (5),
lesdites lumières de fil guide s'étendent à l'intérieur de la paroi de ballonnet (20), des orifices proximaux aux orifices distaux (15),
lesdits orifices proximaux (16, 17) sont positionnés dans la partie du corps allongé (2) qui se trouve entre son extrémité proximale et les moyens d'expansion (5),
**caractérisé en ce que**:
ledit ballonnet (5) a une partie distale (7) et une partie proximale (8), lesdites parties distale (7) et proximale (8) du ballonnet (5) sont des tiges tronconiques.

2. Dispositif endoluminal (1) selon la revendication 1, dans lequel lesdites lumières fixées à la paroi sont au nombre de deux ou de trois.

3. Dispositif endoluminal (1) selon la revendication 1 ou 2, dans lequel lesdites lumières de fil guide (12, 13) ont des orifices de fil guide distaux (14, 15) espacés le long dudit corps allongé (2).

4. Dispositif endoluminal (1) selon la revendication 3, dans lequel une première lumière de fil guide (12) a ledit orifice de fil guide distal (14) au niveau de l'extrémité libre ou pointe de ladite partie d'extrémité distale (3) du corps allongé (2) et lesdits moyens suiveurs de fil guide (11) comprennent au moins l'une desdites lumières de fil guide dans une position approximativement centrale par rapport auxdits moyens d'expansion, pris en considération en coupe à angle droit par rapport au dispositif endoluminal.

5. Dispositif endoluminal (1) selon la revendication 3 ou 4, dans lequel une seconde lumière de fil guide (13) a ledit orifice de fil guide distal (15) à proximité d'une extrémité distale des moyens d'expansion (5).

6. Dispositif endoluminal (1) selon la revendication 3 ou 4, dans lequel une seconde lumière de fil guide (13) a ledit orifice distal (15) dans une partie du corps allongé (2) qui se trouve entre une extrémité distale et une extrémité proximale des moyens d'expansion (5).

7. Dispositif endoluminal (1) selon la revendication 6, dans lequel ledit orifice de fil guide (15) de ladite seconde lumière de fil guide (13) est prévu à proximité de l'axe central de cette partie du corps allongé (2) qui se trouve entre lesdites extrémités distale et proximale des moyens d'expansion (5).

8. Dispositif endoluminal (1) selon l'une quelconque des revendications précédentes, dans lequel lesdites lumières de fil guide (12, 13) ont des orifices de fil guide proximaux (16, 17) positionnés dans des parties du corps allongé (2) positionné, par rapport aux moyens d'expansion (5), au niveau de l'extrémité opposée à son extrémité distale (9).

9. Dispositif endoluminal (1) selon la revendication 8, dans lequel lesdits orifices proximaux (16, 17) sont positionnés au niveau de la pointe proximale de la partie proximale (4) du corps allongé (2).

10. Dispositif endoluminal (1) selon l'une quelconque des revendications précédentes, dans lequel des parties des lumières de fil guide de ladite au moins une paire de lumières de fil guide (12, 13) s'étendent en parallèle à l'intérieur du corps allongé (2).

11. Dispositif endoluminal (1) selon l'une quelconque des revendications précédentes, dans lequel au moins une partie de ladite au moins une paire de lumières de fil guide (12, 13) forme une seule lumière de fil guide.

12. Dispositif endoluminal (1) selon la revendication 3, dans lequel l'orifice de fil guide distal (15) d'une seconde lumière de fil guide (13) est positionné à proximité d'une extrémité proximale des moyens d'expansion (5).

13. Dispositif endoluminal (1) selon la revendication 1, dans lequel les lumières de fil guide (12, 13) ont des orifices distaux (14, 15) positionnés à proximité d'une extrémité proximale des moyens d'expansion (5).
